Europäisches Patentamt

European Patent Office

Office européen des brevets

(19)

(11) Numéro de publication: **0 197 827
B1**

(12) FASCICULE DE BREVET EUROPEEN

(45) Date de publication du fascicule du brevet:
17.05.89

(51) Int. Cl.⁴: **A61B 6/04**

(21) Numéro de dépôt: 86400586.3

(22) Date de dépôt: 19.03.86

(54) Table d'examen à guidage linéaire d'un panneau d'examen.

(30) Priorité: **22.03.85 FR 8504336**

(43) Date de publication de la demande:
**15.10.86 Bulletin 86/42**

(45) Mention de la délivrance du brevet:
**17.05.89 Bulletin 89/20**

(84) Etats contractants désignés:
**DE IT NL**

(56) Documents cités:
**EP-A- 0 077 447
FR-A- 1 378 176
FR-A- 2 403 780
GB-A- 675 065**

(73) Titulaire: **GENERAL ELECTRIC CGR SA., 13, Square
Max-Hymans, F-75015 Paris(FR)**

(72) Inventeur: **Chambron, Edmond, THOMSON-CSF
SCPI 19, avenue de Messine, F-75008 Paris(FR)**

(74) Mandataire: **Ballot, Paul Denis Jacques et al, Cabinet
Ballot-Schmit 84, avenue Kléber, F-75116 Paris(FR)**

**Description**

TLa présente invention concerne une table d'examen à guidage linéaire d'un panneau d'examen, utilisable dans le domaine de la radiologie en général et notamment dans le domaine du radiodiagnostic.

L'exploration radiologique d'un patient peut exiger que celui-ci soit soumis à des examens frontaux ou latéraux, de la tête aux pieds, et dans ce cas un déplacement longitudinal du panneau d'examen supportant le patient, facilite la poursuite de ces examens.

Les panneaux d'examen proprement dit présentent, vis à vis des rayonnements ionisants auxquels est soumis le patient, vis à vis des rayonnements X par exemple, une absorption faible et homogène ; ces conditions étant facilement conservées dans le cas des examens frontaux.

Mais pour les examens latéraux, et en particulier dans les cas où l'axe du faisceau de rayonnement présente un angle égal ou inférieur à 45° par rapport au plan du panneau d'examen, il existe un problème qui réside dans une discontinuité de l'absorption du rayonnement auquel est soumis le patient. Cette discontinuité d'absorption est créée par les bords longitudinaux du panneau, lesquels dans l'art antérieur sont munis de moyens de guidage, généralement métalliques, afin de comporter une bonne rigidité mécanique pour un minimum de matière, et permettant de réaliser le déplacement longitudinal et le guidage du panneau d'examen ; ces moyens de guidage présentant vis à vis dudit rayonnement une absorption très supérieure à celle du ou des matériaux dont est constitué le panneau d'examen.

Une amélioration consiste à munir les bords du panneau d'examen, de moyens de guidage disposés le long d'une moitié seulement de la longueur de ce panneau. L'inconvénient de cette disposition réside en ce qu'elle limite la course du panneau d'examen, et par conséquent la course d'exploration du patient, la longueur du panneau étant elle-même limitée; cette limitation de la longueur du panneau est imposée, outre son coût, par ses caractéristiques de rigidité mécanique, particulièrement quand ce panneau est du type très faiblement absorbant au rayonnement et, que son montage dans la table d'examen l'amène à occuper des positions dans lesquelles il est supporté en porte à faux.

La présente invention concerne une table d'examen à guidage linéaire d'un panneau d'examen, comportant, un bâti par rapport auquel ledit panneau d'examen est déplacé selon un axe longitudinal, des rouleaux d'appui fixés audit bâti et supportant ledit panneau d'examen par une face inférieure de ce dernier, des premier et second moyens de roulement, servant au guidage dudit panneau d'examen, ledit panneau d'examen comportant une partie intérieure réalisée en un premier matériau et enserrée dans une partie extérieure formant enveloppe et réalisée en un second matériau, ladite partie extérieure délimitant ladite face inférieure, une face supérieure et des côtés longitudinaux dudit panneau d'examen, lesdits côtés longitudinaux comportant un longeron servant au guidage dudit panneau d'examen, caractérisé en ce que ladite partie extérieure comporte au moins une partie débordante dépassant par rapport à chacun desdits côtés longitudinaux et dans laquelle est formé ledit longeron, ladite partie débordante étant formée d'une seule pièce avec ladite partie extérieure afin d'être constituée en un même second matériaux et en ce que les premier et second matériaux présentent tous deux une absorption faible vis-à-vis du rayonnement ionisant, de manière à conserver le long desdits côtés longitudinaux une variation d'absorption faible vis-à-vis d'un rayonnement ionisant.

L'invention sera mieux comprise grâce à la description qui suit, faite à titre d'exemple non limitatif, et aux quatre figures annexées parmi lesquelles:

– la figure 1 montre par une vue en perspective, une table d'examen selon l'invention;

– la figure 2 montre, plus en détail par une vue en coupe selon un axe XX représenté à la figure 1, une première version d'une table d'examen conforme à l'invention;

– la figure 3 montre, par une vue de côté, un exemple de réalisation de moyens de guidage;

– la figure 4 montre, par une vue en coupe selon l'axe XX représenté à la figure 1, une version préférée de la table d'examen selon l'invention.

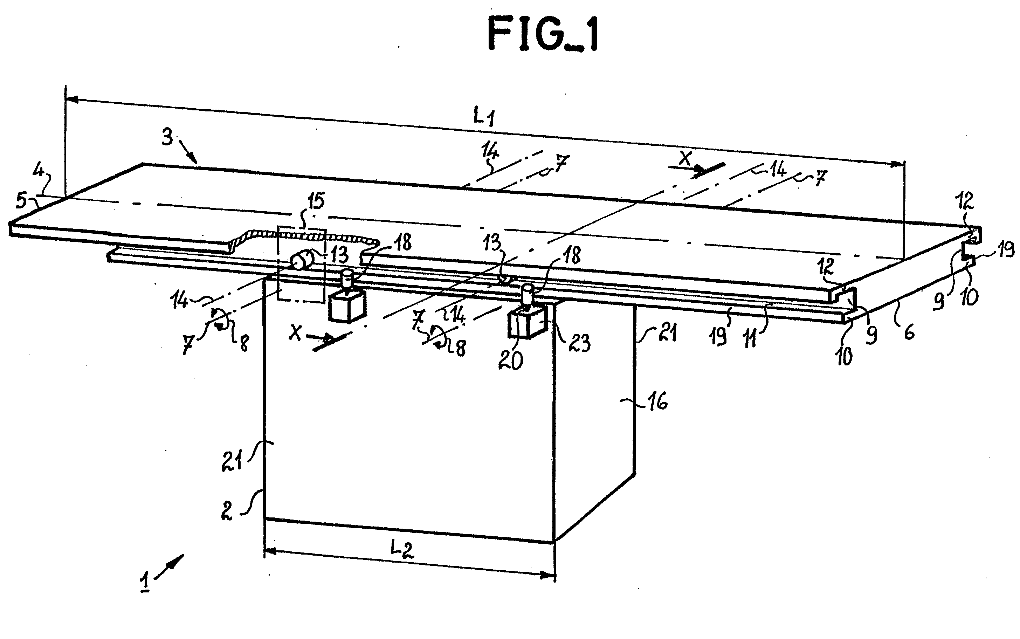

La figure 1 montre par une vue en perspective, une table d'examen 1 radiologique, selon l'invention. La table d'examen 1 comporte un bâti 2 au-dessus duquel est disposé un panneau d'examen 3 destiné à recevoir un patient (non représenté). Le panneau d'examen 3 comporte un axe longitudinal 4, selon lequel il est déplacé par rapport au bâti 2, soit manuellement, soit grâce à des moyens moteurs conventionnels (non représentés), disposés par exemple dans le bâti 2.

Dans l'exemple non limitatif décrit, le panneau d'examen 3 à une longueur L1, supérieur à une longueur L2 du bâti 2, et peut avoir selon sa position par rapport au bâti 2, l'une ou l'autre de ces extrémités 5, 6 en porte à faux ou les deux ainsi que dans l'exemple de la figure 1. Le panneau d'examen 3 repose sur le bâti 2, par l'intermédiaire de rouleaux d'appui, non visibles sur la figure 1, fixés au bâti 2 et disposés selon des axes 7 transversaux à l'axe longitudinal 4; ces rouleaux d'appui étant symbolisés sur la figure 1 par les axes transversaux 7, autour desquels ils sont mis en rotation comme montrés par les flèches 8, dans le déplacement du panneau d'examen 3.

Le panneau d'examen 3 comporte des bords longitudinaux 9, munis chacun d'un longeron 10 comportant une surface de roulement 11 ; les côtés longitudinaux 9 comportant en outre au-dessus des longerons 10 une gouttière de protection 12.

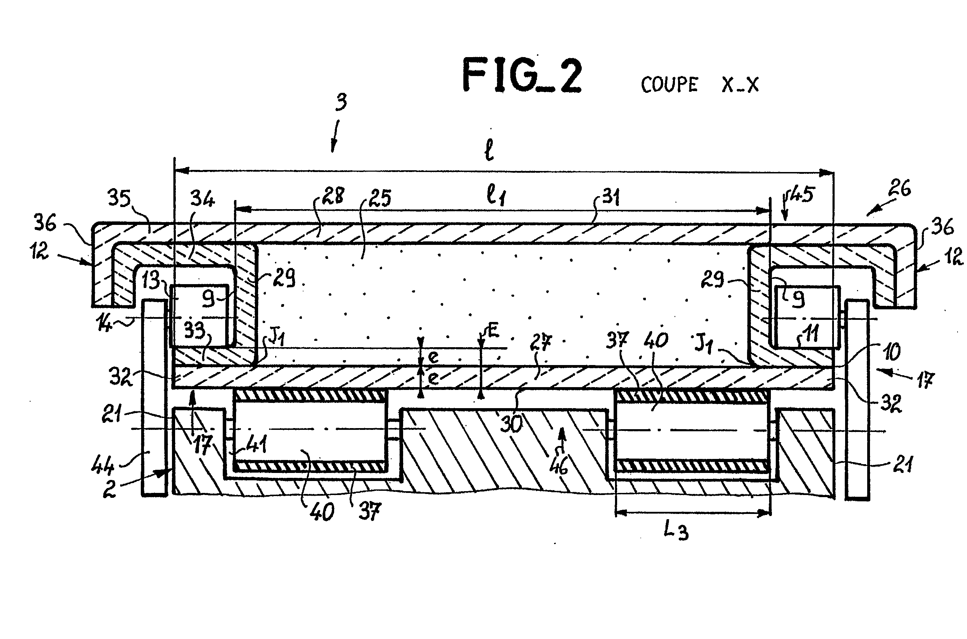

Ainsi qu'il est davantage expliqué dans une suite de la description relative à la figure 2, les longerons 10 sont constitués en un même matériau que l'un des matériaux dans lesquels est réalisé le panneau d'examen 3. Le rôle des longerons 10 est de participer au guidage du panneau d'examen 3 sans créer de discontinuité d'absorption importante sur les côtés longitudinaux 9, vis à vis d'un rayonnement ionisant ou rayonnement X (non représenté) auquel peut être soumis un patient.

La stabilité du panneau d'examen 3 dans son plan, résulte d'une action combinée des rouleaux d'appui précédemment mentionnés, et de premiers moyens de roulement constitués par des roulettes de réaction 13 roulant en appui sur la surface de roulement 11 des longerons 10. Dans l'exemple de la figure 1, deux roulettes de réaction 13 sont visibles, dont une dans un encadré 15 est visible entièrement grâce à une ouverture pratiquée dans le dessin de la gouttière 12. Ces roulettes 13 sont disposées selon de seconds axes transversaux 14 et sont fixées au bâti 2, d'une manière non représentée sur la figure 1 pour plus de clarté de cette dernière. De telles roulettes de réaction 13 existent sur chacun des côtés longitudinaux 9, de préférence à proximité des parois transversales 16 du bâti 2 ; au moins une roulette de réaction 13 coopére avec chacun des rouleaux d'appui précédemment mentionnés, pour éviter un basculement du panneau d'examen 3.

Le guidage longitudinal du panneau d'examen 3 est réalisé grâce à de seconds moyens de roulement constitués, dans l'exemple de cette première version de l'invention, par de secondes roulettes 18 roulant en appui sur chacun des bords longitudinaux 19 de chaque longeron 10. Les roulettes 18 comportent un axe 20 par lequel elles sont fixées aux parois longitudinales 21 du bâti 2, par l'intermédiaire d'une pièce de fixation 23 classique ; au moins deux secondes roulettes 18 fixées à chacune des parois latérales 21 du bâti 2 étant nécessaires pour réaliser ce guidage longitudinal du panneau d'examen 3.

La figure 2 montre, par une vue en coupe transversale selon un axe X-X représenté à la figure 1, des éléments caractéristiques de la table d'examen 1 selon la première version de l'invention.

Le panneau d'examen 3 est composé principalement par deux matériaux différents, présentant tous deux une absorption faible vis à vis du rayonnement X. Le premier matériau forme une partie intérieure 25 qui s'étend sensiblement sur toute la longueur L1 (non visible sur la figure 2) du panneau d'examen 3. Cette partie intérieure 25 est faite d'une mousse compact et dure constituée dans l'exemple non limitatif décrit, par une mousse imide polyméthacrylique. La partie intérieure 25 est enserrée, ou prise en sandwich dans une partie extérieure 26, réalisée dans le second matériau. Dans l'exemple non limitatif décrit, ce second matériau est constitué par des tissus de matériaux composites à base de fibres de carbone pré-imprégnés de résine.

Dans l'exemple non limité décrit, la partie extérieure 26 comporte une première et une seconde plaque 27, 28 sensiblement parallèles entre elles, et de troisième plaques 29 ; ces première, seconde et troisième plaques 27, 28, 29, délimitant respectivement une face inférieure 30, une face supérieure 31 et les côtés longitudinaux 9. Selon une caractéristique de l'invention, le longeron 10 servant au guidage du panneau d'examen 3, et éventuellement la gouttière de protection 12, sont obtenus de construction dès la réalisation du panneau d'examen 3. A cette fin, la partie extérieure 26 comporte, par rapport à chacun des côtés longitudinaux 9, une partie débordante 17 dans laquelle est formée le longeron 10 de guidage. Dans l'exemple non limitatif décrit, ces parties débordantes 17 sont constituées à partir de la première

re plaque 27 dont la largeur $\underline{l}$ est supérieure à une largeur l1 du panneau d'examen 3, considérée entre les bords longitudinaux 9 de ce dernier ; la première plaque 27 comportant ainsi sur chaque côté longitudinal 9, un premier prolongement 32. Les première et seconde plaque 27, 28 sont solidarisées entre elles sur les côtés longitudinaux 9, par l'intermédiaire des troisième plaques 29. Dans l'exemple non limitatif décrit, les troisième plaques 29 sont solidarisées à chacune des première et seconde plaques 27, 28, par un procédé classique, de collage à chaud par exemple. Chacune des troisième plaques 29 comporte à cet effet une partie pliée inférieure 33 et une partie pliée supérieure 34, parallèles aux première et seconde plaques 27, 28 avec lesquelles ces parties pliées inférieures et supérieures 33, 34 sont en contact. Plus précisément, la partie pliée inférieure 33 est solidarisée au premier prolongement 32 de la première plaque 27, c'est-à-dire du côté de la face inférieure 30, et la partie pliée supérieure 34 est réunie à un second prolongement 35 provenant d'un prolongement, sur les côtés longitudinaux 9, de la seconde plaque 28.

Dans cette configuration, chaque longeron 10 est constitué, selon son épaisseur E, par le premier prolongement 32 provenant de la première plaque 27, et par une première partie pliée inférieure 33 obtenue à partir des troisième plaques 29 ; ces premiers prolongement 32 et ces parties pliées inférieures 33 ajoutant leur épaisseur e. Les seconds prolongement 35 réalisés à partir de la seconde plaque 28 et, les parties pliées supérieures 34 réalisées à partir des troisième plaques 29 ajoutent également leur épaisseur (non représentés), et s'étendent vers l'extérieur du panneau d'examen 3 et au-dessus du longeron 10, et sont en outre coudés, de manière à comporter un côté latéral 36 avec lequel elles constituent les gouttières de protection 12.

Le panneau d'examen 3 est supporté par les rouleaux d'appui 40 précédemment mentionnés. Dans l'exemple non limitatif de la description, les rouleaux d'appui 40 sont semi-encastrés dans des logements 41 que comporte le bâti 2, et auquel ils sont fixés selon les axes transversaux 14, par des moyens classiques non représentés pour plus de clarté de la figure. Les rouleaux d'appui 40 comportent une longueur d'appui L3 sur lesquels le panneau d'examen 3 est au moins partiellement en appui sur sa surface inférieure 30 ; les rouleaux d'appui 40 peuvent également s'étendre, d'une manière classique non représentée), selon toute la largeur l1 du panneau d'examen 3.

Selon une caractéristique utile à accroître les performances de la table d'examen 1, il est important ainsi que dans l'exemple non limitatif décrit, que la longueur d'appui L3 des rouleaux d'appui 40 portent le panneau d'examen 3, dans une zone correspondant à une première jonction J1 entre les côtés longitudinaux 9 et la première plaque 27 formant la surface inférieure 30 ; cette disposition permettant de bénéficier de la plus grande rigidité mécanique que comporte le panneau d'examen 3 le long de ces côtés longitudinaux 9, c'est-à-dire grâce aux troisièmes plaques 29.

Les rouleaux d'appui 40 comportent à leur périphérie, une partie souple 37 telle que du caoutchouc par exemple, permettant de mieux absorber les variations d'épaisseur E du longeron 10 ; ceci notamment dans le cas où chaque rouleau d'appui 40 coopère avec une unique roulette de réaction 13. Ainsi qu'il a été précédemment mentionné, les roulettes de réaction 13 roulent sur des bandes de roulement 11, constituées dans l'exemple non limitatif décrit, par les premier prolongement 33 des plaques de côté 29. Les roulettes de réaction 13 sont fixées au bâti 2, par l'intermédiaire d'un étrier 44, et exercent sur les longerons 10, et par conséquent sur le panneau d'examen 3, une poussée dont le sens 45 est opposé au sens 46 d'une poussée exercée sur le panneau d'examen 3 par les rouleaux d'appui 40 ; ceci permettant d'éviter le basculement du panneau d'examen 3 et réalisant le guidage de ce dernier dans son plan.

Les éventuelles variations d'épaisseur E du longeron 10 peuvent être compensés indépendamment de la partie souple 37 dont sont revêtus les rouleaux d'appui 40, en utilisant en coopération avec chaque rouleau d'appui 40, deux roulettes de réaction 13.

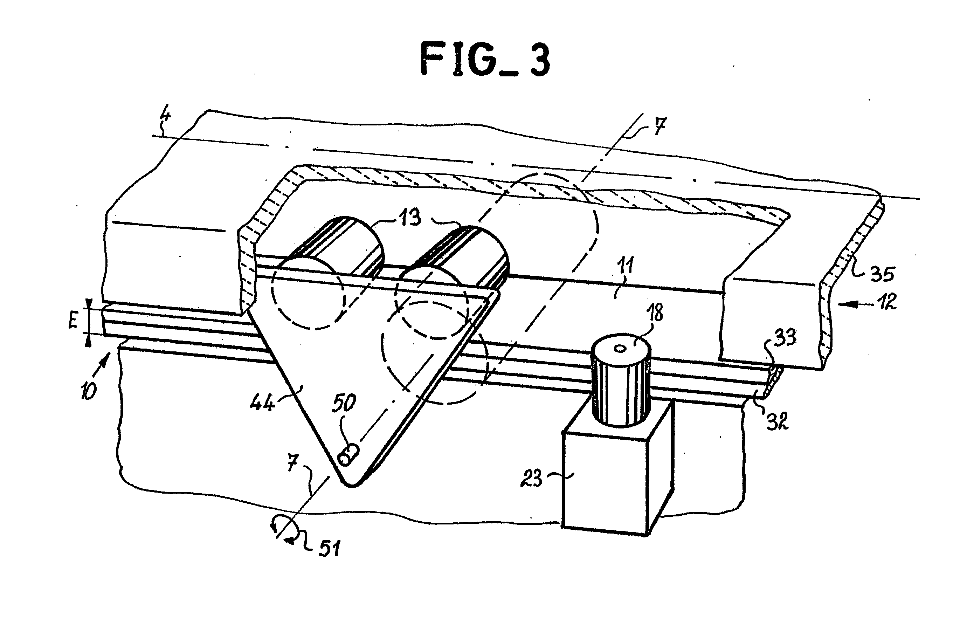

La figure 3 montre un tel montage, par une vue en perspective, correspondant à l'encadré 15 montré sur la figure 1, et dans laquelle la gouttière de protection 12 a été coupée de manière à ne pas masquer les roulettes de réaction 13. Les roulettes de réaction 13 sont fixées par deux à l'étrier 44, lequel étrier est lui-même fixé au bâti 2 par l'intermédiaire d'un pivot 50, autour duquel il peut tourner comme montré par la quatrième flèche 51 ; dans l'exemple non limitatif décrit, le pivot 51 coïncide sensiblement avec l'axe transversal 7 selon lequel est fixé le rouleau d'appui 40, ce dernier étant représenté en traits pointillés. Les roulettes de réaction 13 étant en appui sur la bande de roulement 11 sur laquelle elles roulent dans le déplacement du panneau d'examen 3, leur position peut suivre des variations de l'épaisseur E du longeron 10, grâce au pivotement de l'étrier 50.

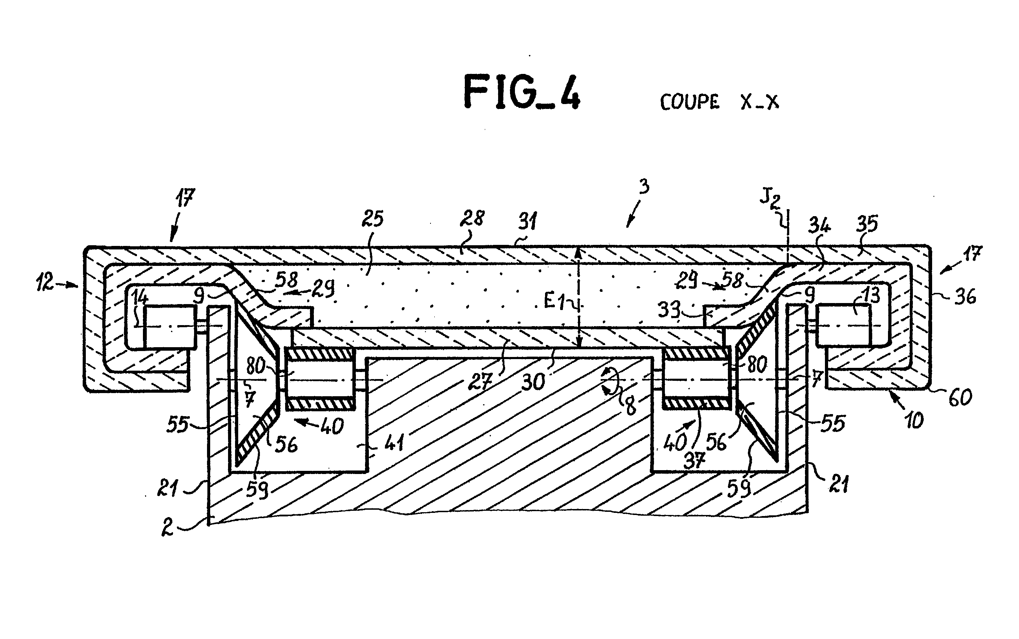

La figure 4 montre, par une vue en coupe selon l'axe XX, une version préférée de l'invention, dans laquelle le panneau d'examen 3 est en appui sur les rouleaux d'appui 40 par sa face inférieure 30 ainsi que dans l'exemple précédent, et en appui en outre par ses côtés longitudinaux 9, de manière à utiliser encore mieux la rigidité mécanique apportée par les troisième plaques 29.

Dans l'exemple non limitatif décrit, les rouleaux d'appui 40 sont constitués par une première partie formant un rouleau cylindrique 80 et par une deuxième partie formant une partie cônique 56. La première partie 80 et la partie cônique 56 sont disposées selon le même axe transversal 7 selon lequel la partie cônique 56 est en dépassement par rapport à la face inférieure 30 du panneau d'examen 3 ; une extrémité 55 de la partie cônique 56, opposée à la première partie 80, c'est-à-dire située vers l'extérieur du bâti 2, constitue la base de la partie cônique 56. La première partie 80 et la partie cônique 56 sont montées d'une manière en elle-même connue (non représenté) de façon à tourner autour d'un axe transversal 7 selon la flèche 8, d'une manière indépendante l'une de l'autre ; leur vitesse de rotation étant différente.

Le panneau d'examen 3 est ainsi supporté par les rouleaux d'appui 40 non seulement par sa face inférieur 30, mais en plus par ses côtés longitudinaux 9 ; ces derniers étant en appui sur une génératrice 59 de la partie cônique 56, les côtés longitudinaux 9 étant à cette fin rendus sensiblement parallèles à ladite génératrice.

Dans l'exemple non limitatif décrit, les parties pliées inférieures 33 des troisième plaques 29 viennent en recouvrement avec la première plaque 27 à laquelle elles sont solidarisées, à l'intérieur du panneau d'examen 3. Les troisième plaques 29 sont conformées pour comporter une partie intermédiaire 58 inclinée et sensiblement parallèle à la génératrice 59 du cône 56. Ces parties intermédiaires inclinées constituent les côtés longitudinaux 9 et s'étendent vers l'extérieur du panneau d'examen 3 pour rejoindre la seconde plaque 28 formant la face supérieure 31, à laquelle elles sont solidarisées. A partir d'un second point de jonction J2 représenté par une ligne en traits pointillés, la troisième plaque 29 et la seconde plaque 28 dépassent des côtés longitudinaux 9 pour former le long de ces derniers la partie débordante 17.

Dans l'exemple de cette réalisation de l'invention, la partie débordante 17 est formée par le second prolongement 35 de la seconde plaque 28 et par la partie pliée supérieure 34 obtenue à partir de la troisième plaque 29 ; la partie débordante 17 étant ensuite pliée pour former le côté latéral 36, sensiblement parallèle à l'épaisseur E1 du panneau d'examen 3, de manière à obtenir la gouttière de protection 12 précédemment mentionnée. La partie débordante 17 est en outre prolongée, à une extrémité inférieure 60 du côté latéral 36, lequel est coudé de manière à retourner vers la bâti 2, et former une partie plane sensiblement parallèle au plan du panneau d'examen 3. Cette partie plane constitue le longeron 10 de guidage sur lequel roulent les roulettes de réaction 13, afin de réaliser le guidage du panneau d'examen 3 dans son plan, en coopération avec les rouleaux d'appui 40.

Il est à remarquer que cette configuration, outre qu'elle permet de supporter le panneau d'examen 3 par sa face inférieure 30 et par ses côtés longitudinaux 9, réalise aussi le guidage longitudinal du panneau d'examen 3, et permet de remplacer les secondes roulettes 18 servant à cet effet dans l'exemple précédent. Le guidage longitudinal est obtenu dans cette version de l'invention, grâce à chacun des côtés longitudinaux 9 en appui sur les parties côniques 56 des rouleaux d'appui 40 ; ces parties côniques 56 assurant ainsi la fonction des seconds moyens de roulement et la fonction de support du panneau d'examen 3.

Une table d'examen 1 selon l'invention, comportant un panneau d'examen 3 déplaçable selon la longueur L1 par rapport au bâti 2, présente par rapport à une table d'examen de même type selon l'art antérieur, notamment les avantages suivants :
- le panneau d'examen 3 est constitué, sur toute sa longueur L1, en matériaux présentant une absorption faible et homogène vis à vis des rayonnements ionisants, notamment le long de ses côtés longitudinaux 9, lesquels côtés longitudinaux comportent des longerons de guidage 10 réalisés en un même second matériau que la table d'examen 3, de manière à ne pas compromettre cette homogénéîté d'absorption le long de ses côtés longitudinaux 9 ;

- les longerons 10 de guidage sont réalisés dans des parties débordantes 17 formées d'une seule pièce avec la partie extérieure 26 du panneau d'examen 3 ; ces parties débordantes 17 étant obtenues de construction, à la réalisation du panneau d'examen 3, elles représentent par rapport à l'art antérieur une simplification de montage et une réduction importante des coûts ;

- l'agencement de la table d'examen 1 selon l'invention, permet en outre d'accroître une course de déplacement du panneau d'examen 3, c'est-à-dire de conférer à la seconde longueur L2 du bâti 2 une dimension très inférieure à la longueur L1 du panneau d'examen 3 et des longerons 10 de guidage, grâce notamment à ce que les côtés longitudinaux 9 sont eux-mêmes en appui sur les rouleaux d'appui 40 ;

- il est à remarquer que l'agencement de la table d'examen 1 selon l'invention permet en outre de réaliser, également de construction, c'està-dire avec des coûts faibles, des gouttières de protection 12 constituées d'une pièce ou non avec les longerons 10 de guidage.

**Revendications**

1. Table d'examen à guidage linéaire d'un panneau d'examen, comportant, un bâti (2) par rapport auquel ledit panneau d'examen (3) est déplacé selon un axe longitudinal (4), des rouleaux d'appui (40) fixés audit bâti (2) et supportant ledit panneau d'examen (3) par une face inférieure (30) de ce dernier, des premier et second moyens de roulement (13, 18, 56), servant au guidage dudit panneau d'examen (3), ledit panneau d'examen (3) comportant une partie intérieure (25) réalisée en un premier matériau et enserrée dans une partie extérieure (26) formant enveloppe et réalisée en un second matériau, ladite partie extérieure (26) délimitant ladite face inférieure (30), une face supérieure (31) et des côtés longitudinaux (9) dudit panneau d'examen (3), lesdits côtés longitudinaux comportant un longeron (10) servant au guidage dudit panneau d'examen (3), caractérisé en ce que ladite partie extérieure (26) comporte au moins une partie débordante (17) dépassant par rapport à chacun desdits côtés longitudinaux (9) et dans laquelle est formé ledit longeron (10), ladite partie débordante (17) étant formée d'une seule pièce avec ladite partie extérieure (26) afin d'être constituée en un même second matériau et en ce que les premier et second matériaux présentent tous deux une absorption faible vis-à-vis du rayonnement ionisant, de manière à conserver le long desdits côtés longitudinaux (9) une variation d'absorption faible vis-à-vis d'un rayonnement ionisant.

2. Table d'examen selon la revendication 1, caractérisé en ce que ladite partie débordante (17) est conformée pour comporter en outre une gouttière de protection (12) réalisée d'une seule pièce avec ledit longeron (10).

3. Table d'examen selon la revendication 1, caractérisé en ce que ladite partie extérieure (26) comporte en outre une seconde partie débordante (12) formée d'une seule pièce avec ladite partie extérieure (26) et dépassant par rapport auxdits côtés longitudinaux (9), de manière à constituer le long de ce dernier une gouttière de protection (12) vis-à-vis du longeron (10).

4. Table d'examen selon l'une des revendications précédentes, caractérisée en ce que ledit panneau d'examen (3) comporte une longueur (L1) supérieure à une seconde longueur (L2) dudit bâti (2), ledit panneau d'examen (3) étant supporté en porte-à-faux selon ledit axe longitudinal (4).

5. Table d'examen selon l'une des revendications précédentes, caractérisée en ce que ladite partie extérieure (26) comporte une première et une seconde plaques (27, 28), formant respectivement ladite face inférieure (30) et ladite face supérieure (31), lesdites première et seconde plaques (27, 28) étant solidarisées par l'intermédiaire de troisième plaques (29), formant lesdits côtés longitudinaux (9), et en ce que des jonctions (J1) entre lesdites première et troisième plaques (27, 29) sont en appui sur lesdits rouleaux d'appui (40).

6. Table d'examen selon l'une des revendications précédentes, caractérisée en ce que lesdits premiers moyens de roulement sont constitués par des roulettes de réaction (13) solidarisées audit bâti (2) et roulant sur lesdits longerons (10), chacun desdits rouleaux d'appui (40) coopérant avec au moins une roulette de réaction (13) exerçant sur ledit longeron (10) une poussée de sens (45) opposé au sens (46) d'une poussée exercée par lesdits rouleaux d'appui (40) sur ledit panneau d'examen (3), de manière à assurer la stabilité dudit panneau d'examen (3) dans son plan.

7. Table d'examen selon la revendication précédente, caractérisée en ce que chaque rouleau d'appui (40) coopère avec deux roulettes de réaction (13) montées sur un même étrier (44), ledit étrier (44) étant solidarisé audit bâti (2) par un pivot (50) autour duquel ledit etrier (44) peut effectuer un mouvement de rotation (51) en fonction de variation d'épaisseur (E) dudit longeron (10).

8. Table d'examen selon l'une des revendications précédentes, caractérisée en ce que lesdits longerons (10) de guidage comportent des bords extérieurs longitudinaux (19) coopérant avec de secondes roulettes (18), afin de réaliser le guidage longitudinal dudit panneau d'examen (3), lesdites secondes roulettes (18) constituant lesdits seconds moyens de roulement.

9. Table d'examen selon les revendications 3 et 5, caractérisée en ce que ladite partie débordante (17) est formée à partir de la première plaque (27) par un premier prolongement (32), et à partir des troisième plaques (29) par une partie pliée inférieure (33), ledit premier prolongement (32) et ladite partie inférieure pliée (33) étant assemblées de manière à ajouter leur épaisseur (e).

10. Table d'examen selon la revendication 5, caractérisée en ce que ladite seconde partie débordante (12) est formée à partir de la seconde plaque (28) par un second prolongement (35) de cette dernière et à partir desdites troisième plaques (29) par une partie supérieure pliée (34), lesdits seconds prolongement (35) et lesdites parties pliées supérieures (34) étant assemblées de manière à ajouter leur épaisseur.

11. Table d'examen selon la revendication 2, caractérisée en ce que lesdits rouleaux d'appui (40) comportent une première partie (80) et une partie cônique (56), ladite partie cônique (56) étant en dépassement par rapport à ladite surface inférieure (30) et en ce que ledit panneau d'examen (3), est supporté par lesdits rouleaux (40) par sa surface inférieure (30) en appui sur ladite première partie (80) et par lesdits côtés longitudinaux (9) en appui sur ladite partie cônique (56), lesdits côtés longitudinaux (9) étant sensiblement parallèles à une génératrice (59) de ladite partie cônique (56).

12. Table d'examen selon la revendication précédente, caractérisée en ce que lesdits second moyens de roulement sont constitués par lesdites parties côniques (56), chacun desdits côtés longitudinaux (9) coopérant avec ladite partie cônique (56) pour obtenir le guidage longitudinal dudit panneau d'examen (3).

13. Table d'examen selon les revendications 2 et 5, caractérisée en ce que ladite première partie débordante (17) est formée à partir de ladite seconde plaque (28) par un second prolongement (35) de cette dernière, et à partir desdites troisième plaques (29) par une partie supérieure pliée (34), ledit second prolongement (35) et ladite partie supérieure pliée (34) étant assemblée d'une manière à ajouter leur épaisseur (e) pour former ladite gouttière de protection (12) et ledit longeron (10) de guidage.

14. Table d'examen selon l'une des revendications précédentes, caractérisée en ce que lesdites parties intérieures et extérieures (25, 26) sont réalisées respectivement en une mousse imide polyméthacrylique, et par des tissus de matériaux composites à base de fibres de carbone pré-imprégnés de résine.

**Patentansprüche**

1. Untersuchungstisch mit linearer Führung einer Untersuchungsplatte mit einem Aufbau (2), in bezug auf welchen diese Untersuchungsplatte (3) entlang einer Längsachse (4) verschoben werden kann, mit Stützrollen (40), welche an diesem Aufbau (2) befestigt sind und diese Untersuchungsplatte (3) über eine untere Fläche (30) derselben tragen, sowie mit ersten und zweiten Rollmitteln (13, 18, 56), welche zur Führung dieser Untersuchungsplatte (3) dienen, wobei diese Untersuchungsplatte (3) einen Innenteil (25) umfaßt, der aus einem ersten Material gebildet ist und in einen als Hülle ausgebildeten und aus einem zweiten Material bestehenden Außenteil (26) eingepreßt ist, wobei dieser Außenteil (26) diese untere Fläche (30), eine obere Fläche (31) sowie Längsseiten (9) dieser Untersuchungsplatte (3) begrenzt und wobei diese Längsseiten einen Längsträger (10) umfassen, der zur Führung dieser Untersuchungstafel (3) dient, dadurch gekennzeichnet, daß dieser Außenteil (26) wenigstens einen überstehenden Teil (17) umfaßt, der in bezug auf jede dieser Längsseiten (9) hinausragt und aus welchem dieser Längsträger (10) ausgebildet ist, wobei dieser überstehende Teil (17) einteilig mit dem Außenteil (26) ausgebildet ist, damit er aus einem selben zweiten Material besteht, und daß das erste sowie das zweite Material eine in bezug auf die ionisierende Strahlung geringe Absorption aufweisen, so daß entlang dieser Längsseiten (9) eine in bezug auf eine ionisierende Strahlung geringe Absorptionsschwankung erhalten bleibt.

2. Untersuchungstisch nach Anspruch 1, dadurch gekennzeichnet, daß der überstehende Teil (17) so ausgebildet ist, daß er ferner eine Schutzrinne (12) umfaßt, welche mit diesem Längsträger (10) einstückig ausgebildet ist.

3. Untersuchungstisch nach Anspruch 1, dadurch gekennzeichnet, daß der Außenteil (26) ferner einen zweiten überstehenden Teil (12) umfaßt, welcher mit diesem Außenteil (26) einteilig ausgebildet ist und in bezug auf die Längsseiten (9) hinausragt, so daß entlang diesen eine Schutzrinne (12) gegenüber dem Längsträger (10) gebildet wird.

4. Untersuchungstisch nach einem der vorstehenden Ansprüche, dadurch gekennzeichnet, daß die Untersuchungsplatte (3) eine Länge (L1) aufweist, welche größer als eine zweite Länge (L2) des Aufbaus (2) ist, wobei diese Untersuchungsplatte (3) entlang der Längsachse (4) auskragend gehaltert ist.

5. Untersuchungstisch nach einem der vorstehenden Ansprüche, dadurch gekennzeichnet, daß der Außenteil (26) eine erste Platte (27) sowie eine zweite Platte (28) umfaßt, wobei die eine die untere Fläche (30) und die andere die obere Fläche (31) bildet, diese erste Platte (27) sowie diese zweite Platte (28) über dritte Platten (29) fest verbunden sind, welche die Längsseiten (9) bilden, und daß Verbindungsstellen (J1) zwischen dieser ersten Platte (27) und diesen dritten Platten (29) sich auf diese Stützrollen (40) abstützen.

6. Untersuchungstisch nach einem der vorstehenden Ansprüche, dadurch gekennzeichnet, daß die ersten Rollmittel aus Gegenröllchen (13) bestehen, welche mit dem Aufbau (2) fest verbunden sind und auf den Längsträgern (10) rollen, wobei jede dieser Stützrollen (40) mit wenigstens einem Gegenröllchen (13) zusammenarbeitet, das auf diesen Längsträger (10) eine Schubkraft ausübt, deren Richtung (45) der Richtung (46) einer durch diese Stützrollen (40) auf diese Untersuchungsplatte (3) ausgeübten Schubkraft entgegengesetzt ist, so daß die Stabilität dieser Untersuchungsplatte (3) in ihrer Ebene gewährleistet wird.

7. Untersuchungstisch nach dem vorstehenden Anspruch, dadurch gekennzeichnet, daß jede Stützrolle (40) mit zwei auf einem selben Bügel (44) befestigten Gegenröllchen (13) zusammenarbeitet, wobei dieser Bügel (44) mit diesem Aufbau (2) über einen Schwenkzapfen (50) fest verbunden ist, um welchen herum dieser Bügel (44) in Abhängigkeit der Veränderung der Dicke (E) dieses Längsträgers (10) eine Drehbewegung (51) ausführen kann.

8. Untersuchungstisch nach einem der vorstehenden Ansprüche, dadurch gekennzeichnet, daß die Führungs-Längsträger (10) längsverlaufende Außenränder (19) umfassen, welche mit zweiten Röllchen (18) zusammenarbeiten, um die Längsführung dieser Untersuchungsplatte (3) zu verwirklichen, wobei diese zweiten Röllchen (18) diese zweiten Rollmittel bilden.

9. Untersuchungstisch nach den Ansprüchen 3 und 5, dadurch gekennzeichnet, daß der überstehende Teil (17) ausgehend von der ersten Platte (27) durch eine erste Verlängerung (32) und ausgehend von den dritten Platten (29) durch einen unteren umgeklappten Teil (33) gebildet ist, wobei diese erste Verlängerung (32) sowie dieser untere umgeklappte Teil (33) so zusammengefügt sind, daß sie sich in ihrer Dicke (e) addieren.

10. Untersuchungstisch nach Anspruch 5, dadurch gekennzeichnet, daß der zweite überstehende Teil (12) ausgehend von der zweiten Platte (28) durch eine zweite Verlängerung (35) dieser letzteren und ausgehend von den dritten Platten (29) durch einen oberen umgeklappten Teil (34) gebildet ist, wobei diese zweite Verlängerung (35) sowie dieser obere umgeklappte Teil (34) so zusammengefügt sind, daß sie sich in ihrer Dicke (e) addieren.

11. Untersuchungstisch nach Anspruch 2, dadurch gekennzeichnet, daß die Stützrollen (40) einen ersten Teil (80) sowie einen kegelförmigen Teil (56) umfassen, wobei dieser kegelförmige Teil (56) in bezug auf die untere Fläche (30) übersteht, und daß die Untersuchungsplatte (3) von diesen Rollen (40) über ihre untere Fläche (30), welche sich auf den ersten Teil (80) abstützt, sowie von den Längsseiten (9), welche sich auf diesen kegelförmigen Teil (56) abstützen, getragen ist, wobei diese Längsseiten (9) zu einer Erzeugenden (59) dieses kegelförmigen Teils (56) im wesentlichen parallel sind.

12. Untersuchungstisch nach dem vorstehenden Anspruch, dadurch gekennzeichnet, daß die zweiten Rollmittel durch die kegelförmigen Teile (56) gebildet sind, wobei jede der Längsseiten (9) mit diesem kegelförmigen Teil (56) zusammenarbeitet, um die Längsführung dieser Untersuchungsplatte (3) zu bewirken.

13. Untersuchungstisch nach den Ansprüchen 2 und 5, dadurch gekennzeichnet, daß der erste überstehende Teil (17) ausgehend von der zweiten Platte (28) durch eine zweite Verlängerung (35) der letzteren und ausgehend von diesen dritten Platten (29) durch einen oberen umgeklappten Teil (34) gebildet ist, wobei diese zweite Verlängerung (35) sowie dieser obere umgeklappte Teil (34) so zusammengebaut sind, daß sie sich in ihrer Dicke (e) addieren, um die Schutzrinne (12) sowie den Längsträger (10) zu bilden.

14. Untersuchungstisch nach einem der vorstehenden Ansprüche dadurch gekennzeichnet, daß der Innenteil (25) aus einem Imid-Polymethacrylschaum und der Außenteil (26) aus Stoffen oder Gewirken von Verbundmaterialen besteht, die auf mit Harz vorimprägnierten Kohlenstoff-Fasern basieren.

**Claims**

1. An examination table with linear guidance means of an examination panel, comprising a frame (2) in relation to which the said examination panel (3) is desplaced along a longitudinal axis (4), support rollers (40) fixed to the said frame (2) and supporting the said examination panel (3) by way of a lower face (30) of the latter, first and second rolling means (13, 18 and 56) serving to guide the said examination panel (3), the said examination panel (3) comprising a lower part (25) made of a first material and held within an outside part (26) forming an envelope and made of a second material, the said outer part (26) delimiting the said lower face (30), and an upper face (31) and longitudinal sides (9) of the said examination panel (3), the said longitudinal sides (9) comprising a longitudinal member (10) serving for guiding of the said examination panel (3), characterized in that the said outer part (26) comprises at least one overlapping part (17) extending beyond each of these longitudinal sides (9) and in which the said longitudinal member (10) is formed, the said overlapping part (17) being formed integrally with the said outer part (26) in order to be constituted of the same second material and in that the first and the second materials each have a low degree of absorption for ionizing radiation in such a manner as to have a small variation of absorption for ionizing radiation along the said longitudinal sides (9).

2. The examination table as claimed in claim 1, characterized in that said overlapping part (17) is shaped so as to also have a protection gutter (12) made integrally with the said longitudinal member (10).

3. The examination table as claimed in claim 1, characterized in that the said outer part (26) furthermore comprises a second overlapping part (12) made integrally with the said outer part (26) and overlapping in relation to the said longitudinal sides (9) in such a manner as to constitute a protection gutter (12) along the latter opposite to the longitudinal member (10).

4. The examination table as claimed in any one of the preceding claims, characterized in that the said examination panel (3) has a length (L1) greater than a second length (L2) of the said frame (2), said examination panel (3) being supported in an overhanging manner along the said longitudinal axes (4).

5. The examination table as claimed in any one of the preceding claims, characterized in that the said outer part (26) comprises a first and a second plate (27a and 28) respectively forming the said lower face (30) and the said upper face (31), the said first and second plates (27 and 28) being attached by the intermediary of second plates (29), forming the said longitudinal sides (9) and in that the junctions between the first and the third plates (27 and 29) are supported on the said supporting rollers (40).

6. The examination table as claimed in any one of the preceding claims, characterized in that the said first rolling means are constituted by reaction rollers (13) attached to the said frame (2) and running on the said longitudinal members (10), each of these support rollers (40) cooperating with at least one reaction roller (13) exercising on the said longitudinal member (10) a thrust in a direction (45) opposite to the direction (46) of a thrust exercised by the said support rollers (40) on the said examination panel (3) in such a manner as to ensure the stability of the said examination panel (3) in its plane.

7. The examination table as claimed in the preceding claim, characterized in that each support roller (40) cooperate with two reaction rollers (13) mounted on the same stirrup (44), the said stirrup (44) being attached to the frame (2) by a pivot (50) around which the said stirrup (44) may move in rotation (51) as a function of the variation in thickness (E) of the said longitudinal member (10).

8. The examination table as claimed in any one of the preceding claims, characterized in that the said longitudinal members (10) for guiding comprise outer longitudinal edges (19) cooperating with second rollers (18) in order to effect longitudinal guidance of the examination panel (3), the said second rollers (18) constituting the said second rolling means.

9. The examination table as claimed in claims 3 and 5, characterized in that the said overlapping part (17) is formed by the second plate (27) in the form of a first prolongation (32) and by the third plates (29) in the form of an inner flanged part (33), the said first prolongation (32) and the said inner flanged part (33) being assembled in such a manner as to add their thicknesses (e) together.

10. The examination table as claimed in claim 5, characterized in that the second overlapping part (12) is formed by the second plate (28) in the form of a second prolongation (35) of the latter and by the third plates (29) in the form of an upper flanged part (34), the said second prolongations (35) and the said upper flanged parts (34) being assembled in such a manner as to add their thicknesses together.

11. The examination table as claimed in claim 2, characterized in that the said support rollers (40) comprise a first part (80) and a conical part (56), the said conical part (56) being arranged to overlap the said inner surface (30) and in that the said examination panel (3) is supported by the said rollers (40) by way of its lower surface (30) supported on the said first part (80) and by the said longitudinal sides (9) supported on the said conical part (56), the said longitudinal sides (9) being substantially parallel to a generatrix (59) of the said conical part (56).

12. The examination table as claimed in the preceding claim, characterized in that the said second rolling means are constituted by the said conical parts (56), each of the said longitudinal sides (9) cooperating with the said conical part (56) in order to effect the longitudinal guidance of the said examination panel (3).

13. The examination table as claimed in claims 2 and 5, characterized in that the said first overlapping part (17) is formed by the second plate (28) in the form of a second prolongation (35) of the latter, and by the said third plates (29) in the form of an upper flanged part (34), the said second prolongation (35) and the said upper flanged part (34) being assembled in such a manner as to add their thicknesses (e) together in order to form the said protective gutter (12) and the said guiding longitudinal member (10).

14. The examination table as claimed in any one of the preceding claims, characterized in that the said lower and outer parts (25 and 26) are respectively made of a polymethacrylic imide foam and by composite material fabrics based on resin prepegs with carbon fibers.

# FIG_1

EP 0 197 827 B1

# FIG_2 COUPE X_X

EP 0 197 827 B1

# FIG_ 3

EP 0 197 827 B1

# FIG_4    COUPE X_X

EP 0 197 827 B1